# EUROPÄISCHE PATENTSCHRIFT

(11) **EP 2 453 229 B1**
(45) Veröffentlichungstag und Bekanntmachung des Hinweises auf die Patenterteilung: **09.05.2018**
(21) Anmeldenummer: 11188648.7
(22) Anmeldetag: 10.11.2011
(51) Int. Cl.: G01N 27/404, G01N 33/49

(54) **Sauerstoffsensor mit mikroporöser Elektrolytschicht und teiloffener Deckmembran**
Oxygen sensor with a microporous electrolyte layer and partially open cover membrane
Capteur d'oxygène avec une couche d'électrolyte microporeux et une membrane de couverture partiellement ouverte

(30) Priorität: 10.11.2010 EP 10190756
(43) Veröffentlichungstag der Anmeldung: 16.05.2012
(73) Patentinhaber: F. Hoffmann-La Roche AG, 4070 Basel (CH); Roche Diagnostics GmbH, 68305 Mannheim (DE)
(72) Erfinder: Offenbacher, Helmut, 8020 Graz (AT)
(74) Vertreter: Weickmann & Weickmann PartmbB

(56) Entgegenhaltungen:
- WO-A1-01/36957
- WO-A1-2009/090094
- US-A- 4 933 048

## Beschreibung

Die vorliegende Erfindung betrifft einen elektrochemischen Sensor zur Bestimmung von in einem wässrigen Messmedium gelöstem Sauerstoff, ein Verfahren zu dessen Herstellung, sowie ein Verfahren zur Bestimmung von in einem wässrigen Messmedium gelöstem Sauerstoff unter Verwendung des elektrochemischen Sensors.

Sensoren zur Analytik von Körperflüssigkeiten stellen wichtige Bestandteile klinisch relevanter Analyseverfahren dar. Hierbei steht insbesondere eine schnelle und präzise Messung von Analyten im Vordergrund, welche die Bestimmung sogenannter "Point-of-Care"-Parameter gestattet. Point-of-Care-Untersuchungen werden vor allem auf Intensivstationen und in der Anästhesie, aber auch in Ambulanzen durchgeführt. Zu diesen sogenannten Notfallparametern zählen unter anderem die Blutgaswerte (und hier im Besonderen der Sauerstoffgehalt und Kohlendioxidgehalt), der pH-Wert, Elektrolytwerte, sowie gewisse Metabolitenwerte.

Point-of-Care-Untersuchungen haben den Vorteil, dass die Ergebnisse bereits nach kurzer Zeit vorliegen, da zum einen der Transport der Proben zu einem spezialisierten Labor entfällt und zum anderen keine Rücksicht auf die zeitlichen Abläufe des Labors genommen werden muss. Um eine möglichst zeitnahe Bestimmung von Notfallparametern zu ermöglichen, sollten hierfür eingesetzte Sensoren, welche für eine mehrmalige Analytbestimmung konzipiert sind, grundsätzlich eine hohe "in-use"-Lebensdauer aufweisen, um die Zeitspanne zwischen turnusmäßigen Sensorwechseln, welche beispielsweise nach Ablauf einer vorgegebenen Maximalbenutzungsdauer oder nach einer vorgegebenen Anzahl von Messungen erforderlich sind, zu vergrößern. Hierdurch werden die durch den Sensorwechsel bedingten Zeiten, in welchen ein solcher Point-of-Care-Analysator nicht einsatzfähig ist, d.h. für Messungen nicht zur Verfügung steht, reduziert. Darüber hinaus sollte die einem Sensorwechsel unmittelbar folgende Sensoraktivierungszeit, welche die Zeitspanne vom Sensorwechsel bis zur Einsatzfähigkeit des neuen Sensors für analytische Messungen angibt, möglichst gering gehalten werden.

Zur Durchführung derartiger Point-of-Care-Untersuchungen können beispielsweise Teststreifen oder auch medizinische Analysatoren mit Multi-Use-Sensoren verwendet werden, wodurch der manuelle Aufwand für die Durchführung auf ein Minimum reduziert wird. Sensoren für einen Point-of-Care-Einsatz werden in der Regel nahezu vollständig automatisiert in entsprechend eingerichteten analytischen Messgeräten betrieben und erfordern von der Probenvorbereitung bis zur Bereitstellung des Testergebnisses oft nur wenige und einfache Eingriffe des Benutzers. Sie können sowohl für eine einzige als auch für eine wiederholte Messung der zu bestimmenden Parameter ausgebildet sein.

Die Messung von Sauerstoff bzw. dessen Partialdruck (pO₂) in einem wässrigen Messmedium kann beispielsweise unter Verwendung amperometrischer Sensoren, umfassend mindestens eine Arbeitselektrode und mindestens eine Gegenelektrode, erfolgen. Bei Elektroden vom Clark-Typ trennt in der Regel eine gasdurchlässige und weitgehend ionen- und flüssigkeitsundurchlässige Membran den Probenraum räumlich vom Innenelektrolytraum ab. Der Innenelektrolytraum ist mit einer Elektrolytlösung befüllt, in welcher sich eine Arbeitselektrode und eine Gegenelektrode befinden. Eine dem derzeitigen Stand der Technik entsprechende Elektrodenanordnung mit einer mikroporösen Innenelektrolytschicht ist in WO 2009/090094 A1 beschrieben.

Ein wichtiges Kriterium bei der Entwicklung und Bereitstellung elektrochemischer Sensoren für Point-of-Care-Untersuchungen sind deren spezielle Anforderungen an die Messbedingungen, insbesondere an das zumeist nur geringe zur Verfügung stehende Probenvolumen. So stehen zur Bestimmung von Notfallparametern im Allgemeinen nur geringe Probenmengen (z.B. 100 µl oder weniger) zur Verfügung. Soll mit geringen Probenmengen eine Vielzahl von Parametern bestimmt werden, müssen die einzelnen Elektroden sowie deren Abstände zueinander möglichst klein sein.

Ein weiteres wesentliches Kriterium bei der Entwicklung und Bereitstellung elektrochemischer Sensoren für Point-of-Care-Untersuchungen ist, wie vorstehend erwähnt, deren Lebensdauer. Hierbei besteht das Bedürfnis, sowohl eine dauerhafte Lagerfähigkeit vor Inbetriebnahme des Sensors als auch eine hohe "in-use"-Lebensdauer des elektrochemischen Sensors zu erzielen.

Um eine dauerhafte Lagerfähigkeit zu gewährleisten, sollten die in dem elektrochemischen Sensor befindlichen Elektroden trocken, d.h. im Wesentlichen wasserfrei, gelagert und erst unmittelbar vor Inbetriebnahme mit dem flüssigen Innenelektrolyten in Kontakt gebracht werden. Zu diesem Zweck kann der flüssige Innenelektrolyt beispielsweise "in situ" gebildet werden, indem der trockene Sensor zur Inbetriebnahme mit einer wässrigen Lösung in Kontakt gebracht wird, wobei Wasser in den Innenelektrolytraum eindiffundiert und dort befindliche Ionen unter Bildung des flüssigen Innenelektrolyten löst.

Um eine hohe "in-use"-Lebensdauer von beispielsweise wenigstens 500 Messungen oder 3 bis 4 Wochen zu erreichen, wie dies für moderne für Point-of-Care-Analysatoren erwünscht wird, müssen die verschiedenen Schichten des elektrochemischen Sensors ferner kompatibel zueinander sein. So dürfen sie sich insbesondere nicht voneinander ablösen oder Risse ausbilden, wie dies beispielsweise durch Quellung hervorgerufen werden kann.

Schließlich spielt, wie vorstehend erläutert, auch die schnelle Aktivierbarkeit eines erstmals für analytische Messungen in einen Analysator eingesetzten elektrochemischen Sensors eine wichtige Rolle. Insofern sollte, um eine zeitnahe Bestimmung von Analyten in einer Probe zu ermöglichen, eine möglichst kurze Zeitspanne zwischen dem Einsetzen eines neuen elektrochemischen Sensors in einen Analysator und dessen Einsatzfähigkeit für analytische Messungen liegen.

EP 0 805 973 B1 offenbart eine Vorrichtung sowie ein Verfahren zum Messen der Konzentration von Gasen in einer Probe. Als Vorrichtung dient ein elektrochemischer Sensor, welcher eine Arbeitselektrode, eine Gegenelektrode, eine Elektrolytschicht und eine gaspermeable Membran umfasst, wobei die Elektrolytschicht aus einem fotogeformten proteinösen Gallert besteht. Um eine frühzeitige Kontamination der negativ polarisierten, aus Gold bestehenden Arbeitselektrode (Kathode) durch positiv geladene, von der aus Silber bestehenden Gegenelektrode (Anode) stammende Silberionen zu vermeiden, muss der Abstand der beiden durch eine Gallertschicht in elektrischem Kontakt stehenden Elektroden mindestens 1 mm betragen.

US 5,387,329 beschreibt einen planaren elektrochemischen Sauerstoffsensor, bei dem ein quellbares Polymer, dessen Quellrate unter dem Zweifachen seines Trockenvolumens liegt, als hygroskopischer Elektrolyt verwendet wird und hierbei eine hydrophile, für Wasser und Kationen permeable Elektrolytschicht ausbildet. Ein im Rahmen dieses Dokuments bevorzugtes quellbares Polymer ist Nafion®, ein sulfoniertes Tetrafluorethylenpolymer, dessen mit Lithium beladene Sulfonatgruppen dem Polymer ionomere Eigenschaften verleihen und einen Austausch von Lithiumionen gegen Silberionen bewirken. Dies hat den Effekt, dass bei einem amperometrischen Sauerstoffsensor mit Silber-Gegenelektrode die effektive Wanderungsgeschwindigkeit der Silberionen zur Arbeitselektrode erniedrigt wird.

Die in EP 0 805 973 B1 und US 5,387,329 verwendeten Elektrolytschichten sind mit Nachteilen behaftet. So ist beispielsweise die Herstellung sehr dünner Schichten (ca. 1 µm) quellfähiger Polymere (wie z.B. die in EP 0 805 973 B1 fotogeformten proteinösen Gallerte) sehr aufwendig.

Weiterhin führen die beim Betrieb eines Sauerstoffsensors freigesetzten Silberionen bei sehr dünnen Schichten und sehr kleinen Elektrolytvolumina schnell zu Störsignalen sowie zum Anstieg des Messsignals und des Nullpunktes, wobei der Nullpunkt den Strom angibt, der bei 0% Sauerstoff in der Probe über den Sauerstoffsensor fließt. Bei Verwendung dickerer, quellender Schichten (ca. 10-50 µm) in einem mehrschichtigen Aufbau wird andererseits die Bildung von Undichtigkeiten im Schichtaufbau gefördert. Hierdurch bereitet es Schwierigkeiten, die angestrebte Langlebigkeit des Sensors zu erreichen.

Ein bedeutender Nachteil dünner, wasserhaltiger Polymerschichten ist darüber hinaus, dass störende Silberionen nach Anlegen eines elektrischen Feldes zwischen den beiden Elektroden auf direktem Weg durch das Polymer wandern und so nach relativ kurzer Betriebsdauer zur Arbeitselektrode gelangen und diese kontaminieren, wodurch die effektive Nutzungsdauer derartiger, vorzugsweise planarer Sauerstoffsensoren deutlich begrenzt wird.

WO 2009/090094 A1 offenbart einen elektrochemischen Sensor, bei dem die oben beschriebenen Nachteile teilweise beseitigt sind. Zu diesem Zweck enthält der elektrochemische Sensor eine zwischen der Arbeitselektrode und der Gegenelektrode lokalisierte Elektrolytschicht, welche mindestens ein partikelförmiges Material und mindestens ein Bindemittel umfasst. Hierbei bilden das partikelförmige Material, bei welchem es sich vorzugsweise um ein Alumosilikat wie beispielsweise ein Zeolith handelt, und das Bindemittel eine mikroporöse Schicht aus, die alsdann von einem flüssigen Elektrolyten durchsetzt werden kann.

Im Unterschied zu einer gelartigen Elektrolytschicht, wie sie beispielsweise in EP 0 805 973 B1 beschrieben ist, müssen an der Gegenelektrode eines in EP 0 805 973 B1 beschrieben ist, müssen an der Gegenelektrode eines solchen elektrochemischen Sensors generierte Silberionen auf ihrem Weg zur Arbeitselektrode durch ein Labyrinth aus Mikroporen wandern, wodurch die Silberionen in ihrer Wanderungsgeschwindigkeit gebremst werden. Weiterhin sieht WO 2009/090094 A1 in einer bevorzugten Variante die Möglichkeit vor, dass das partikelförmige Material zusätzlich lonenaustauschergruppen aufweist, an welche die Silberionen zumindest temporär binden können. Durch diese beiden Maßnahmen kann die Lebensdauer des elektrochemischen Sensors auf etwa 3 bis 4 Wochen verlängert werden.

US 4,933,048 offenbart einen elektrochemischen Sensor zur potentiometrischen Bestimmung der Konzentration von Ionen in einem wässrigen Messmedium, umfassend (a) eine Arbeitselektrode und eine Referenzelektrode, (b) eine über der Referenzelektrode angeordnete Elektrolytschicht, und (c) eine zumindest teilweise über der Elektrolytschicht angeordnete wasserpermeable und ionenimpermeable Deckschicht.

Die der Erfindung zugrunde liegende Aufgabe bestand somit darin, einen elektrochemischen Sensor zur Bestimmung von Sauerstoff (im folgenden auch als (gasförmiger) Analyt bezeichnet) bereitzustellen, bei welchem weitere Nachteile des Standes der Technik zumindest teilweise beseitigt sind. Insbesondere sollte der Sensor neben einer dauerhaften Lagerfähigkeit und einer hohen "in-use"-Lebensdauer eine kurze Aktivierungszeit bei erstmaligem Einsatz gewährleisten. Weiterhin sollte der Sensor einfach und kostengünstig hergestellt werden können.

Diese Aufgabe wurde erfindungsgemäß gelöst mittels eines elektrochemischen Sensors zur Bestimmung von in einem wässrigen Messmedium gelöstem Sauerstoff, entsprechend Anspruch 1. Die Arbeitselektrode des erfindungsgemäßen elektrochemischen Sensors kann aus einem beliebigen Material bestehen, welches dem Fachmann für die Zwecke der vorliegenden Erfindung geeignet erscheint. Die Arbeitselektrode ist beispielsweise aus Gold oder aus einem Verbundmaterial auf Basis von Gold ausgebildet. Denkbar sind indessen auch andere Metalle wie beispielsweise Silber. Demgegenüber ist die Gegenelektrode aus Silber oder aus einem Verbundmaterial auf Basis von Silber ausgebildet.

Die elektrochemischen Sensoren der vorliegenden Erfindung umfassen weiterhin eine Elektrolytschicht, welche zwischen der Arbeitselektrode und der Gegenelektrode angeordnet ist. Die Elektrolytschicht ist dabei aus mindestens einem partikelförmigen Material und mindestens einem Bindemittel gebildet, welche zusammen eine poröse, nicht-quellbare Gerüststruktur ausbilden, deren Poren sich zwischen den einzelnen Partikeln des partikelförmigen Materials befinden. Im Vergleich zu einer reinen Polymerschicht bzw. Gallert- oder Gelschicht erhöht das auf diese Weise gebildete Porenlabyrinth die Wegstrecke, welche von den sich zwischen den Elektroden bewegenden Ionen zu durchlaufen ist, und erhöht somit die Lebensdauer des Sensors.

Partikelförmige Materialien, welche in den elektrochemischen Sensoren der vorliegenden Erfindung Anwendung finden, können anorganischer oder organischer Natur sein und umfassen insbesondere dichte anorganische Materialien oder dichte (weichmacherfreie) organische Polymere. Bevorzugte anorganische partikelförmige Materialien umfassen Controlled Porous Glass (CPG), Kieselgele, Silikate und Alumosilikate (auch Aluminosilikate genannt), welche vornehmlich aus der Gruppe bestehend aus Gerüstsilikaten und Schichtsilikaten ausgewählt sind. Organische Polymere, welche erfindungsgemäß als partikelförmiges Material eingesetzt werden können, umfassen beispielsweise Mikropartikel aus Polystyrol, Polyamid, Polyvinylchlorid, Polyvinylidenchlorid, Poly(meth)acrylat, Polyacrylnitril, sowie Copolymere hiervon, sind jedoch nicht auf diese beschränkt.

Als vorteilhaft hat sich im Rahmen der Erfindung die Verwendung eines natürlich vorkommenden oder synthetischen Alumosilikats, stärker bevorzugt eines synthetischen Alumosilikats, als partikelförmiges Material erwiesen. Der Begriff "synthetisches Alumosilikat", wie er im Rahmen der vorliegenden Erfindung verwendet wird, umfasst sowohl vollsynthetische Alumosilikate als auch Alumosilikate, welche durch künstliche Veränderung (z.B. chemisch) eines natürlich vorkommenden Alumosilikats erhalten werden. Beispiele für natürlich vorkommende oder synthetische Alumosilikate umfassen Feldspäte, Glimmer, Mullit, Sillimanit und Zeolithe, sind jedoch nicht auf diese beschränkt.

In einer bevorzugten Variante des erfindungsgemäßen elektrochemischen Sensors weist das partikelförmige Material Kanäle, insbesondere Kanäle mit einem Durchmesser zwischen etwa 0.1 nm bis etwa 10 nm, und optional lonentauschergruppen auf. In einer stärker bevorzugten Ausführungsform handelt es sich bei dem partikelförmigen Material um ein Zeolith, wie beispielsweise Faujasit, Chabasit, Mordenit oder Natrolith, in welchem Polyeder, Schichten oder Ketten aus eckenverknüpften [(Al,Si)O₄]-Tetraedern vorliegen, die ein von Kanälen durchzogenes, anionisches Raumnetzwerk ausbilden.

Je nach Art des Zeoliths weisen die Kanäle einen Durchmesser von etwa 0.5 nm bis etwa 5.0 nm, bevorzugt von etwa 0.7 nm bis etwa 2.0 nm, auf und enthalten an ihren inneren und äußeren Oberflächen lonentauschergruppen, insbesondere Kationentauschergruppen. Die Verwendung von Zeolithen mit innerer Kanalstruktur und lonentauschergruppen hat sich insbesondere bei amperometrischen Elektroden vom Clark-Typ als besonders vorteilhaft erwiesen. Besonders bevorzugt umfasst der erfindungsgemäße Sensor Faujasit, am stärksten bevorzugt Faujasit-Na, als partikelförmiges Material.

Infolge ihrer kanalhaltigen Ausgestaltung weisen Zeolithe eine große innere Oberfläche auf, welche mit einem flüssigen Elektrolyten in Kontakt treten kann. Da andererseits Ionen geeigneter Größe, welche in der Elektrolytflüssigkeit enthalten sind, durch die Kanäle des Zeolithen wandern können, kann die Menge an unerwünschten Ionen reduziert werden. So tritt bei elektrochemischen Sensoren, welche silberhaltige Gegenelektroden enthalten, insbesondere das Problem auf, dass mit Inbetriebnahme des Sensors Silberionen aus der Gegenelektrode freigesetzt werden, in Richtung der Arbeitselektrode wandern und sich unter Passivierung der Arbeitselektrode als elementares Silber auf dieser absetzen.

Ähnliche Vorgänge laufen auch bei anderen als Elektrodenmaterialien eingesetzten Metallen wie beispielsweise Blei ab, welches bei Kontakt mit der Elektrolytflüssigkeit Bleiionen freisetzen kann, die ebenfalls als Störkationen wirken können. Durch Verwendung eines elektrochemischen Sensors gemäß der vorliegenden Erfindung kann die effektive Wegstrecke zwischen Gegenelektrode und Arbeitselektrode vergrößert und damit die Gefahr einer raschen Passivierung der Arbeitselektrode verringert werden, was sich in einer höheren Lebensdauer des Sensors äußert.

Andererseits können bestimmte partikelförmige Materialien, wie beispielsweise Zeolithe, aufgrund ihrer anionischen Gerüststruktur auch als Ionentauscher wirken. So können beispielsweise an der Gegenelektrode freigesetzte und zur Arbeitselektrode wandernde Silberionen von Zeolithen absorbiert und gegen geeignete, weniger kritische Kationen, wie etwa Natriumionen, ausgetauscht werden, wodurch die Menge an freien Silberionen im Elektrolyten reduziert wird und einer Passivierung der Arbeitselektrode entgegen gewirkt werden kann, was ebenfalls zu einer höheren Lebensdauer des elektrochemischen Sensors führt.

Die Größe der Partikel des partikelförmigen Materials kann entsprechend den jeweiligen Anforderungen variiert werden. Im Rahmen der vorliegenden Erfindung weisen die Partikel des partikelförmigen Materials üblicherweise einen mittleren Partikeldurchmesser von etwa 0.1 µm bis etwa 10 µm auf, wobei ein mittlerer Partikeldurchmesser von etwa 1.0 µm bis etwa 5.0 µm bevorzugt ist. In jedem Fall sollte die Partikelgröße des porösen Materials stets kleiner sein als die Schichtdicke der Elektrolytschicht, welche im Bereich von etwa 5 µm bis etwa 30 µm, bevorzugt im Bereich von etwa 10 µm bis etwa 20 µm, liegt.

Neben dem partikelförmigen Material umfasst die Elektrolytschicht als weitere Komponente mindestens ein Bindemittel. Bevorzugt handelt es sich bei dem Bindemittel um ein organisches Bindemittel, wie beispielsweise ein vernetztes oder unvernetztes, lineares oder verzweigtes organisches Polymer. Stärker bevorzugt umfasst das in der Elektrolytschicht enthaltene organische Bindemittel ein beliebiges vernetztes oder unvernetztes synthetisches Polymer, welches vorzugsweise aus der Gruppe bestehend aus einem Phenolharz, einem Epoxidharz, einem Vinylharz, einem PVC-Copolymer, einem Zweikomponenten-Epoxidharz, einem Polyurethanharzsystem und einem UV-härtbaren Polyacrylat-Momomergemisch ausgewählt ist. Besonders bevorzugt wird als vernetztes oder unvernetztes synthetisches Polymer ein Phenolharz, insbesondere ein vernetztes Phenolharz, eingesetzt.

Das bevorzugt nicht-quellbare Bindemittel ist mengenmäßig so bemessen, dass es die Partikel des anorganischen oder organischen partikelförmigen Materials zu einer porösen, nicht-quellbaren Gerüststruktur verbindet. Dies bedeutet, dass das Bindemittel die Zwischenräume zwischen den Partikeln des partikelförmigen Materials nicht vollständig ausfüllt, so dass sich eine nicht-quellbare Gerüststruktur bildet, welche Poren mit einem Durchmesser von etwa 500 nm bis etwa 5 µm, bevorzugt von etwa 1 µm bis etwa 3 µm, aufweist. Erfindungsgemäß bewirkt die poröse, nicht-quellbare Gerüststruktur eine relative Flüssigkeitsaufnahme von etwa 20 Gew.-% bis etwa 50 Gew.-%, bevorzugt von etwa 26 Gew.-% bis etwa 44 Gew.-%, bezogen auf das Trockengewicht der porösen, nicht-quellbaren Gerüststruktur.

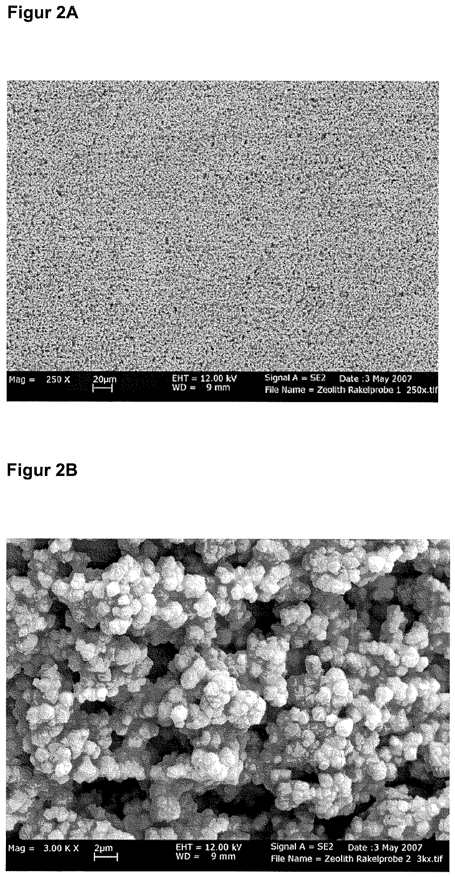

Die erforderlichen Mengen an Bindemittel können in Abhängigkeit von der Art und Menge des verwendeten partikelförmigen Materials sowie der gewünschten Porengröße variieren und von einem Fachmann den jeweiligen Anforderungen angepasst werden. Die Figuren 2A und 2B zeigen jeweils elektronenmikroskopische Aufnahmen der Elektrolytschicht eines solchen Sensors, in welcher Partikel eines partikelförmigen Materials unter Verwendung eines Bindemittels zu einer porösen, nicht-quellbaren Gerüststruktur verbunden sind. Die poröse, nicht-quellbare Gerüststruktur weist eine regelmäßige Verteilung der Poren auf und besteht aus Einheiten, die in der Regel etwa 1 bis 3 Partikel breit und kettenartig verbunden sind.

Erfindungsgemäß kann die Elektrolytschicht neben dem mindestens einen partikelförmigen Material und dem mindestens einen Bindemittel ferner einen oder mehrere Hilfsstoffe umfassen. Hilfsstoffe, welche zu diesem Zweck verwendet werden können, umfassen insbesondere synthetische Cellulosederivate wie beispielsweise Alkylcellulosen, wobei der Begriff "Alkyl" für einen geradkettigen oder verzweigten Kohlenwasserstoffrest mit 1 bis 6 Kohlenstoffatomen steht. Bevorzugte Alkylcellulosen in Sinne der vorliegenden Erfindung sind aus der Gruppe bestehend aus Methylcellulose, Ethylcellulose, Propylcellulose, Ethylmethylcellulose und Propylmethylcellulose ausgewählt. Durch die Zugabe kleiner Mengen dieser Alkylcellulosen kann die Benetzungsfähigkeit der Strukturoberfläche mit dem flüssigen Elektrolyten verbessert werden. Weitere geeignete Hilfsstoffe umfassen beispielsweise Entschäumer.

Der flüssige Elektrolyt, welcher für eine leitende Elektrolytverbindung zwischen der Arbeitselektrode und der Gegenelektrode in dem erfindungsgemäßen elektrochemischen Sensor sorgt, kann ein beliebiger Elektrolyt, z.B. ein Elektrolyt auf Basis von Wasser, sein, welcher beim Anlegen einer Spannung durch gerichtete Bewegung von Ionen einen Transport von Ladungsträgern bewirkt. Im Rahmen der vorliegenden Erfindung ist es bevorzugt, dass der flüssige Elektrolyt ein Alkalimetallchlorid beinhaltet.

Als Alkalimetallchlorid, welches in einem elektrochemischen Sensor gemäß der vorliegenden Erfindung als Leitsalzkomponente des flüssigen Elektrolyten dient, können insbesondere Natrium- oder/und Kaliumchlorid verwendet werden. Die Verwendung von Natrium- oder/und Kaliumchlorid als Hauptkomponente des Elektrolyten bietet den Vorteil, dass die bei der Verwendung silberhaltiger Gegenelektroden aus der Gegenelektrode freigesetzten Silberionen infolge Silberchloridfällung zusätzlich an einer Wanderung in Richtung der Arbeitselektrode gehindert werden, wodurch die Ablagerung von elementarem Silber auf der Arbeitselektrode reduziert werden kann.

In einer bevorzugten Ausführungsform umfasst der flüssige Elektrolyt neben dem Alkalimetallchlorid weiterhin mindestens ein wasserlösliches Polymer. Als wasserlösliches Polymer kann ein beliebiges Polymer verwendet werden, welches durch Einbringen in die vorstehend beschriebene flüssige Elektrolytlösung eine Erhöhung der Viskosität der Flüssigkeit bewirkt und damit ebenfalls zur Reduzierung der Wanderungsgeschwindigkeit freigesetzter Ionen, beispielsweise freigesetzter Silberionen, beitragen kann. Wasserlösliche Polymere, welche im Rahmen der vorliegenden Erfindung Anwendung finden, sind beispielsweise Polyethylenglykol, Polyvinylpyrrolidon (PVP) und Alkylpolyglykoside, wobei die Elektrolytflüssigkeit vorzugsweise Polyethylenglykol oder/und Polyvinylpyrrolidon als wasserlösliches Polymer beinhaltet.

Im Rahmen der vorliegenden Erfindung ist es weiterhin bevorzugt, dass die Elektrolytschicht die nicht-flüchtigen Bestandteile des flüssigen Elektrolyten vor Inbetriebnahme des elektrochemischen Sensors umfasst. Hierzu kann eine diese Bestandteile enthaltende Flüssigkeit, vorzugsweise der flüssige Elektrolyt, z.B. durch Auftropfen in die Poren der nicht-quellbaren Gerüststruktur eingebracht und nach Entfernen eines Überstandes an Elektrolytflüssigkeit, z.B. durch Abtupfen, für einen Zeitraum von mehreren Stunden, z. B. für einen Zeitraum von 48 Stunden oder mehr, bei einer Temperatur > 25°C, z.B. bei einer Temperatur von 65°C, gelagert werden, um ein Abdampfen der flüchtigen Bestandteile des flüssigen Elektrolyten zu ermöglichen. Auf diese Weise kann sichergestellt werden, dass nach dem Trockenschritt die nicht-flüchtigen Bestandteile des Elektrolyten, wie beispielsweise Alkalimetallchloride sowie weitere Substanzen, welche u.a. wasserlösliche Polymere umfassen, in der Elektrolytschicht des elektrochemischen Sensors vorliegen.

Alternativ können die nicht-flüchtigen Elektrolytbestandteile bereits vor Ausbildung der pörosen, nicht-quellbaren Gerüststruktur einem das mindestens eine partikelförmige Material, das mindestens eine Bindemittel und gegebenenfalls weitere Substanzen enthaltenden Gemisch, welches beispielsweise in Form einer Paste vorliegt, zugesetzt werden. Das auf diese Weise erhaltene Gemisch kann im Anschluss ausgehärtet werden, wobei eine poröse, nicht-quellbare Gerüststruktur ausgebildet wird, deren Poren die nicht-flüchtigen Bestandteile des flüssigen Elektrolyten enthalten. Diese Vorgehensweise bietet gegenüber dem oben beschriebenen Verfahren, d.h. gegenüber einer Einbringung des flüssigen Elektrolyten in die Poren einer bereits bestehenden Gerüststruktur, den Vorteil, dass die verschiedenen Bestandteile des flüssigen Elektrolyten einfacher, homogener und besser reproduzierbar in die poröse, nicht-quellbare Gerüststruktur des erfindungsgemäßen Sensors eingebracht werden können.

Die elektrochemischen Sensoren der vorliegenden Erfindung umfassen des weiteren eine über der vorstehend beschriebenen Elektrolytschicht angeordnete gaspermeable Deckschicht, welche mindestens eine Ausnehmung umfasst und damit einen Kontakt eines Teilbereichs der Elektrolytschicht mit dem wässrigen Messmedium ermöglicht. Der Ausdruck "Kontakt eines Teilbereichs der Elektrolytschicht mit dem wässrigen Messmedium" ist im Rahmen der vorliegenden Anmeldung funktionell zu verstehen. Wesentlich ist, dass im Bereich der Ausnehmung der Deckschicht kein Material der Deckschicht selbst vorliegt, d.h. die im Stand der Technik beschriebene durchgängige Deckschicht in diesem Bereich unterbrochen ist und somit die Eigenschaften der Deckschicht, insbesondere deren Gaspermeabilität sowie deren weitestgehende Ionen- und Flüssigkeitsundurchlässigkeit, in diesem Bereich funktionell nicht zur Wirkung kommen.

Im Rahmen der vorliegenden Anmeldung ist unter dem Ausdruck "Kontakt eines Teilbereichs der Elektrolytschicht mit dem wässrigen Messmedium" somit nicht nur ein direkter Kontakt, wie er beispielsweise durch eine spalt- oder lochförmige Ausnehmung in der gaspermeablen Deckschicht hervorgerufen wird, sondern auch ein indirekter Kontakt, wie er beispielsweise durch eine mit einem geeigneten Material verfüllte spalt- oder lochförmige Ausnehmung in der Deckschicht bewirkt wird, zu verstehen. Hierbei ist lediglich von Bedeutung, dass das zur Verfüllung der Ausnehmung verwendete Material nicht mit dem Material der gaspermeablen Deckschicht identisch ist, um nicht wieder eine vollständig geschlossene gaspermeable Deckschicht mit den erwähnten Nachteilen zu erhalten.

Durch die mindestens eine Ausnehmung in der gaspermeablen Deckschicht kann die Aktivierungszeit der elektrochemischen Sensoren bei erstmaligem Gebrauch, was im Fachbereich auch als "wet-up"-Zeit beschrieben wird, gegenüber elektrochemischen Sensoren mit vollständig geschlossener gaspermeabler Deckschicht deutlich reduziert werden, wodurch die erfindungsgemäßen elektrochemischen Sensoren schneller für analytische Messungen einsatzbereit sind. Hierzu werden üblicherweise Referenzflüssigkeiten, wie beisielsweise Kalibrationslösungen oder Konditionierungsflüssigkeiten eingesetzt, welche im Rahmen der vorliegenden Anmeldung den Messmedien funktionell gleichgestellt sind.

Um die Aktivierungszeit eines elektrochemischen Sensors zu evaluieren, kann beispielsweise die Veränderung des für den Sensor gemessenen Standby-Stroms über die Zeit erfasst werden. Als Standby-Strom wird dabei jener Strom bezeichnet, welcher beispielsweise bei einem Sauerstoffsensor bei Anlegen einer Spannung von 700 mV unter Standby-Bedingungen über die Kathode fließt, wobei unter Standby-Bedingungen der Kontakt des Sauerstoffsensors mit einer Kalibrationslösung, deren Sauerstoffgehalt auf den Sauerstoffpartialdruck in der Luft abgestimmt ist, zu verstehen ist.

Der Standby-Strom wird bei einem Sauerstoffpartialdruck pO₂ = 147 mmHg (entspricht dem Umgebungsluft entsprechenden pO₂-Wert einer Kalibrationslösung bei einem barometrischen Druck von ca. 750 mmHg bei 37°C) bestimmt und setzt sich aus 2 Anteilen zusammen:
(i) einem konstanten Anteil, unabhängig vom pO₂-Wert der Kalibrationslösung, und
(ii) einem variablen, vom pO₂-Wert der Kalibrationslösung abhängigen Anteil.

Bei einem idealisierten Sensor ist der konstante Stromanteil null, d.h. bei einem Sauerstoffpartialdruck von 0 mmHg ist der gemessene Strom ebenfalls null. Der konstante, vom pO₂-Wert unabhängige Signalanteil wird als Nullpunktstrom bezeichnet. Bei realen Sensoren ist der Nullpunktstrom jedoch zumeist > 0. Der variable, vom pO₂-Wert abhängige Signalanteil wird als Slope bezeichnet und ist die Stromänderung (in [nA]) bei einer Änderung des Sauerstoffpartialdrucks von 0 auf 147 mmHg.

Ist der Nullpunktstrom > 0 und errechnet man, welchem Sauerstoffwert dieser Strom beim idealisierten Sensor entspricht (d.h. wenn kein konstanter Signalanteil vorhanden wäre), erhält man per Definition den Gas-Nullpunkt in mmHg. Der Gas-Nullpunkt entspricht demnach dem konstanten Signalanteil eines realen Sensors, ausgedrückt als pO₂-Wert (in [mmHg]), wobei zur Berechnung modellhaft angenommen wird, dass der Signalanteil des Sensors bei 0 mmHg tatsächlich null beträgt.

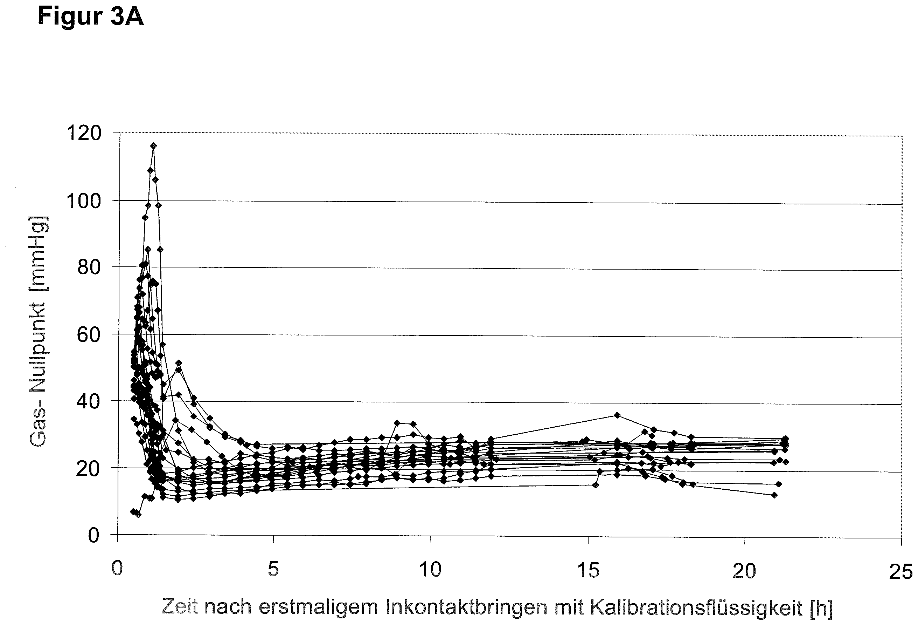

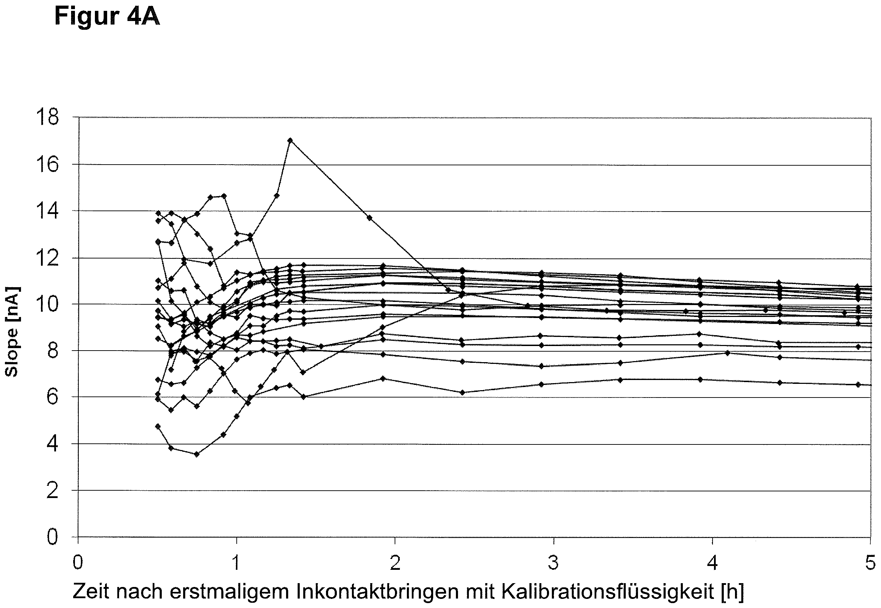

Ein Kollektiv elektrochemischer Sensoren mit vollständig geschlossener gaspermeabler Deckschicht zeigt insbesondere innerhalb der ersten Stunden nach erstmaligem Inkontaktbringen mit einer Kalibrationslösung eine deutliche Abnahme und eine starke Drift des Gas-Nullpunkts (siehe Fig. 3a) und des Slopes (siehe Fig. 4a) bis zum Erreichen des Regelbereichs. Insbesondere zeigen die Messsignale des Kollektivs derartiger elektrochemischer Sensoren in diesem Zeitraum nach erstmaliger Aktivierung des Sensors ein starkes Schwanken und teilweise signifikante Sprünge dieser Größen, infolge dessen das Messsignal mitunter so stark schwankt, dass eine zuverlässige Analytbestimmung in dieser Phase, auch unter Berücksichtigung möglicher Korrekturen des Sensorsignaldrifts, oft nicht möglich ist.

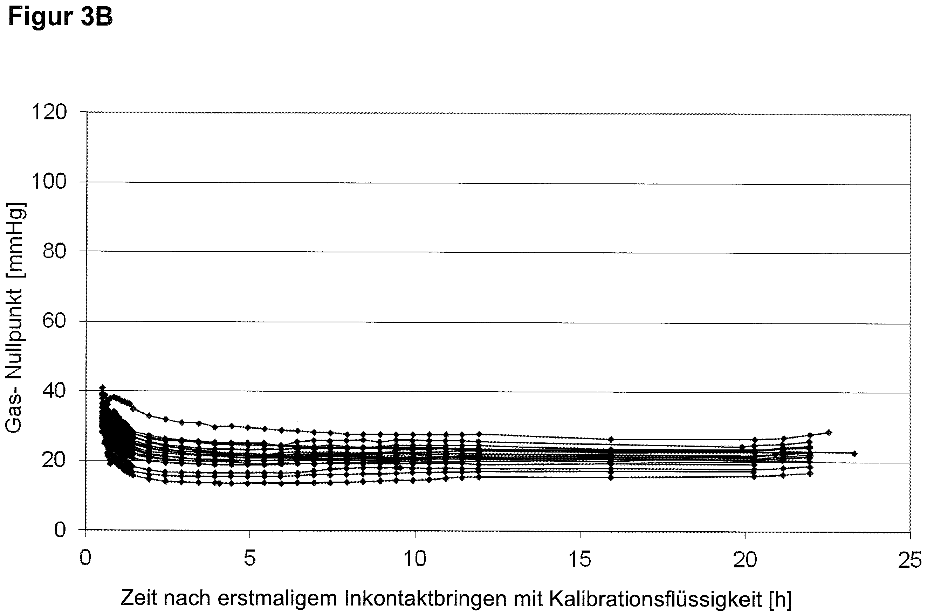

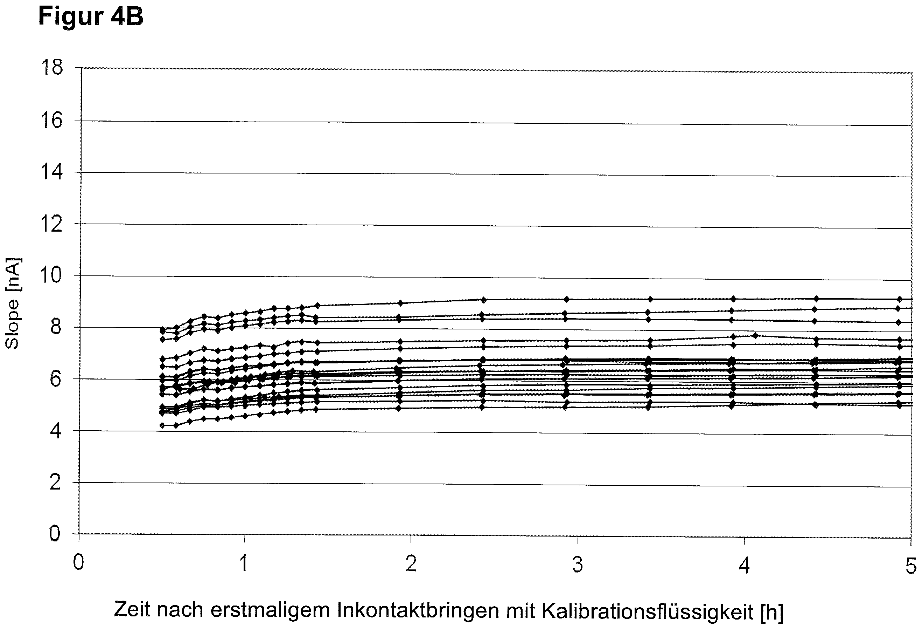

Im Gegensatz hierzu liegen der Gas-Nullpunkt (siehe Fig. 3b) und der Slope (siehe Fig. 4b) eines Kollektivs erfindungsgemäßer elektrochemischer Sensoren mit zumindest teilweise offener Deckmembran bereits zu Beginn der Messung im Regelbereich und zeigen eine innerhalb der Vorgaben liegende Drift ohne die im Stand der Technik teilweise auftretenden starken Schwankungen dieser Größen, wodurch bereits zu früheren Zeitpunkten, auch mit Hilfe zusätzlicher möglicher Korrekturen des Sensorsignaldrifts, zuverlässigere Analytbestimmungen möglich sind.

In den Figuren 3 und 4 ist auf der x-Achse jeweils die Zeit (in [h]) nach dem erstmaligen Inkontaktbringen (t = 0) des (zuvor trocken gelagerten) Sensors mit hierfür vorgesehenen Referenzflüssigkeiten, wie beispielsweise Kalibrationsflüssigkeiten oder Konditionierungsflüssigkeiten, verstanden. Prinzipiell kann der Sensor in analoger Weise aber auch mit anderen Flüssigkeiten, wie beispielsweise Messmedien, aktiviert werden.

Begründbar ist das Phänomen, dass elektrochemische Sensoren mit vollständig geschlossener gaspermeabler Deckschicht zu Beginn der Sensoraktivierungsphase hohe Standby-Ströme liefern, mit im Zuge der Hydratation des Innenelektrolytraums auftretenden Verdrängungen der im Trockenstatus vorhandenen Luft sowie deren Kompression. So kann die Restluft im Innenelektrolytraum einen kürzeren Diffusionsweg des gasförmigen Analyten von der Probe zur Arbeitselektrode bedingen, was zu hohen Strömen führen kann. Durch sukzessiven Abbau der im Innenelektrolytraum enthaltenen Restluft während der "wet-up"-Phase normalisiert sich das Verhalten des elektrochemischen Sensors, bis letztlich ein unter stabilen Betriebsbedingungen erforderlicher gasblasenfreier Innenelektrolytraum vorliegt.

Im Gegensatz hierzu bleibt das Diffusionsverhalten von Sauerstoff in den elektrochemischen Sensoren der vorliegenden Erfindung durch die innerhalb der gaspermeablen Deckschicht enthaltene Ausnehmung wesentlich definierter. Lufteinschlüsse im Innenelektrolytraum, wie sie zu Beginn der Sensoraktivierung auftreten, können über die Querdiffusion in der mikroporösen Elektrolytschicht, insbesondere bei einer hypobaren Sensoraktivierung, besser abgebaut werden.

Darüber hinaus führt die mindestens eine, in der gaspermeablen Deckschicht enthaltene Ausnehmung dazu, dass beispielsweise an der Gegenelektrode gebildete Silberionen in den Probenraum oberhalb des elektrochemischen Sensors abdiffundieren können, was zu einer Verringerung der Konzentration an freien Silberionen in der Elektrolytschicht und damit zu einer Verringerung der Bildung von Silberkeimen und der Fällung von kolloidem Silber innerhalb der porösen, nicht-quellbaren Gerüststruktur führt. Auf diese Weise kann die "in-use"-Lebensdauer der elektrochemischen Sensoren auf deutlich über 4 Wochen angehoben und damit gegenüber den aus dem Stand der Technik bekannten Sensoren weiter verbessert werden.

Die mindestens eine Ausnehmung in der gaspermeablen Deckschicht kann grundsätzlich jede beliebige Größe oder/und Form aufweisen, welche dem Fachmann zum Erreichen einer geringeren Sensoraktivierungszeit geeignet erscheint. Weist die gaspermeable Deckschicht mehrere Ausnehmungen auf, so sind diese vorzugsweise außerhalb des Bereichs der Elektroden angeordnet, wobei die Ausnehmungen gleiche oder unterschiedliche Größe oder/und Form besitzen können. In einer bevorzugten Variante der Erfindung besitzt die mindestens eine Ausnehmung eine Breite im Bereich von etwa 50 µm bis etwa 500 µm, stärker bevorzugt im Bereich von etwa 100 µm bis etwa 300 µm.

Was die geometrische Form betrifft, so ist die mindestens eine Ausnehmung vorzugsweise spaltförmig oder fensterartig ausgebildet, wobei eine spaltförmige Ausnehmung im Sinne der vorliegenden Anmeldung dahingehend zu verstehen ist, dass die gaspermeable Deckschicht in diesem Fall in Form zweier nicht direkt miteinander verbundener, vorzugsweise parallel zueinander angeordneter Teilbereiche vorliegt. Eine fensterartige Ausnehmung bezeichnet demgegenüber jedwede runde, ovale oder eckige Ausnehmung, welche zu keiner Aufteilung der gaspermeablen Deckschicht in mindestens zwei nicht direkt miteinander verbundene Teilbereiche führt.

In Bezug auf die räumliche Anordnung der mindestens einen Ausnehmung sehen die erfindungsgemäßen elektrochemischen Sensoren vor, dass die Ausnehmung grundsätzlich an einer beliebigen Stelle der gaspermeablen Deckschicht ausgebildet sein kann. Bevorzugt ist die mindestens eine Ausnehmung allerdings zumindest teilweise, d.h. teilweise oder vollständig, über dem zwischen der Arbeitselektrode und der Gegenelektrode liegenden Bereich der Elektrolytschicht angeordnet. Eine derartige Anordnung hat den Vorteil, dass überwiegend Silberionen, welche sich in dem zwischen der Arbeitselektrode und der Gegenelektrode liegenden Bereich der Elektrolytschicht befinden, in den Probenraum oberhalb des Sensors abtransportiert werden, wodurch speziell die Fällung von kolloidem Silber innerhalb der zwischen den beiden Elektroden ausgebildeten porösen, nicht-quellbaren Gerüststruktur verringert werden kann.

In den elektrochemischen Sensoren der vorliegenden Erfindung ist die mindestens eine, in der gaspermeablen Deckschicht enthaltene Ausnehmung in einer Ausführungsform unverfüllt. In einer alternativen Ausführungsform besteht die Möglichkeit, dass die Ausnehmung mit einem geeigneten hydrophilen polymeren Füllmaterial verfüllt ist, wobei sich das hydrophile polymere Füllmaterial von dem Material unterscheidet, aus welchem die gaspermeable Deckschicht gebildet ist. Hydrophile polymere Füllmaterialien, welche im Rahmen der vorliegenden Anmeldung zur Anwendung gelangen können, umfassen beispielsweise Hydrogele, wobei Hydrogele aus Polyether-Polyurethan-Copolymeren oder Polyvinylpyrrolidon (PVP) besonders bevorzugt sind.

Die hydrophilen polymeren Füllmaterialien besitzen vorzugsweise eine hohe Permeabilität für niedermolekulare wasserlösliche Substanzen, insbesondere für ionische Substanzen und für Wassermoleküle. Dies ermöglicht einerseits einen (im Vergleich zu vollständig geschlossenen Deckschichten) schnelleren Eintritt von Wasser in den elektrochemischen Sensor während der "wet-up"-Phase. Andererseits wird auf diese Weise, im Gegensatz zu vollständig geschlossenen Deckschichten, das "Abdiffundieren" von Silberionen aus dem elektrochemischen Sensor in den Probenraum ermöglicht, während gleichzeitig das Eindiffundieren von beispielsweise höhermolekularen Blutbestandteilen und Zellen aus der Probe in die Elektrolytschicht verhindert werden kann.

Die erfindungsgemäß ausgebildete gaspermeable Deckschicht erlaubt das Eindringen des (unter Normalbedingungen) gasförmigen Analyten in den elektrochemischen Sensor, verhindert jedoch an sämtlichen Stellen mit Ausnahme der erfindungsgemäß vorhandenen Ausnehmung weitestgehend den Eintritt von insbesondere Ionen oder/und nicht-flüchtigen Bestandteilen des wässrigen Messmediums in den elektrochemischen Sensor.

In einer bevorzugten Ausführungsform ist die gaspermeable Deckschicht derart ausgestaltet, dass sie die Arbeitselektrode vollständig und die Gegenelektrode zumindest teilweise, d.h. teilweise oder vollständig, überdeckt. Der Begriff "überdeckt", wie er im Rahmen der vorliegenden Anmeldung verwendet wird, bedeutet, dass die ausgewählte Elektrode bzw. die sich über der ausgewählten Elektrode befindliche Elektrolytschicht von der gaspermeablen Deckschicht in dem jeweils angegebenen Bereich überzogen ist, so dass in diesem Bereich kein Kontakt zwischen der ausgewählten Elektrode bzw. der sich über der ausgewählten Elektrode befindlichen Elektrolytschicht und dem wässrigen Messmedium erzielt wird.

Im Rahmen der vorliegenden Erfindung hat es sich als besonders vorteilhaft erwiesen, wenn die gaspermeable Deckschicht die Arbeitselektrode in einem Bereich von mindestens 100 µm, stärker bevorzugt in einem Bereich von mindestens 500 µm, über die Abmessungen der Arbeitselektrode hinaus überdeckt. Sofern die gaspermeable Deckschicht die Gegenelektrode lediglich teilweise überdeckt, so ist es bevorzugt, dass mindestens 90% der Gegenelektrode, bezogen auf deren Oberfläche, von der gaspermeablen Deckschicht überdeckt sind. In einer besonders bevorzugten Variante der Erfindung überdeckt die gaspermeable Deckschicht indessen sowohl die Arbeitselektrode als auch die Gegenelektrode vollständig, wodurch eine verbesserte Abschirmung der Elektroden gegenüber Interferenten aus dem Messmedium erzielt werden kann, da letztere nicht auf direktem Weg, d.h. ohne Beteiligung der Elektrolytschicht, zu den Elektroden diffundieren können.

Die gaspermeable Deckschicht kann aus jedem beliebigen Material bestehen, welches dem Fachmann für die oben beschriebenen Zwecke dienlich erscheint. Bevorzugt umfasst die gaspermeable Deckschicht mindestens ein polymeres Material, wobei sich Polysiloxane, Polysulfone oder/und Polytetrafluorethylen als besonders vorteilhaft erwiesen haben. Als Polysiloxane können erfindungsgemäß insbesondere Silikonkautschuke zur Anwendung gelangen, wie sie beispielsweise unter der Handelsbezeichnung DELO-GUM® (Fa. DELO, Deutschland) kommerziell erhältlich sind.

Geeignete Polysulfone umfassen insbesondere Polysulfone mit längeren Alkylketten, wie beispielsweise C₁₆-Alkylketten. Ein derartiges Produkt ist unter dem Handelsnamen BIOBLAND® (Fa. ANATRACE, USA) bekannt.

Die Erzeugung der gaspermeablem Deckschicht kann auf unterschiedliche Weise erfolgen. Ein bevorzugtes Verfahren besteht in der Verwendung vorgefertigter Deckschichten, welche auf den elektrochemischen Sensor aufgebracht werden. Eine Fixierung der Membran auf dem Sensor kann hierbei mittels beliebiger Techniken erfolgen, wobei Verklebung oder Laserverschweißung als bevorzugt anzusehen sind.

Alternativ besteht die Möglichkeit, die gaspermeable Deckmembran *in situ* zu erzeugen, indem eine Lösung eines geeigneten gaspermeablen Polymers oder Prepolymers auf den elektrochemischen Sensor aufgebracht und anschließend getrocknet wird. Das Aufbringen des Polymers auf den elektrochemischen Sensor erfolgt bevorzugt durch Aufsprühen, Tauchcoaten, Dispergieren oder Siebdrucken einer vorzugsweise verdünnten Lösung des Polymers oder Prepolymers, ist jedoch nicht auf diese Verfahren beschränkt. Als Lösungsmittel wird dabei bevorzugt ein organisches Lösungsmittel, insbesondere ein organisches Lösungsmittel mit einem Siedepunkt von ≤ 100°C, eingesetzt, wobei das Lösungsmittel je nach den Eigenschaften des Polymers oder Prepolymers oder/und der Auftragetechnik variiert und vom Fachmann dementsprechend ausgewählt werden kann.

Die auf diese Weise erhaltenen und in einem elektrochemischen Sensor gemäß der vorliegenden Erfindung eingesetzten gaspermeablen Deckschichten besitzen üblicherweise eine Dicke von etwa 5 µm bis etwa 30 µm. In einer bevorzugten Variante weist die gaspermeable Deckschicht eine Dicke von mindestens 30%, insbesondere eine Dicke von 50% bis 100%, der Dicke der erfindungsgemäß verwendeten Elektrolytschicht auf.

Bevorzugt ist der erfindungsgemäße elektrochemische Sensor für mehrfache, d.h. zeitlich nacheinander erfolgende Analytbestimmungen ausgebildet. Dies ist insbesondere in Anwendungen erwünscht, in welchen mit einem Sensor eine Vielzahl von Proben gemessen werden soll. In einer bevorzugten Ausführungsform sieht die Erfindung demnach vor, dass der elektrochemische Sensor als sensitiver Bestandteil einer (Durchfluss-) Messzelle ausgebildet ist, in die eine den Analyten enthaltende Probenflüssigkeit eingebracht wird und welche beispielsweise ein austauschbarer Teil eines Analysators ist.

Der erfindungsgemäße elektrochemische Sensor dient zur Bestimmung von Sauerstoff in einem wässrigen Messmedium, welches aus einer beliebigen Quelle stammen kann. Der Ausdruck "Bestimmung von in einem wässrigen Messmedium gelöstem Sauerstoff", wie er im Rahmen der vorliegenden Anmeldung verwendet wird, umfasst dabei sowohl die Bestimmung des Vorhandenseins bzw. der Konzentration des gelösten Sauerstoffs im wässrigen Messmedium als auch die Bestimmung des Gaspartialdrucks von Sauerstoff im wässrigen Messmedium. In einer bevorzugten Ausführungsform handelt es sich bei dem wässrigen Messmedium um eine Körperflüssigkeit, wobei Vollblut als besonders bevorzugt gilt.

In einem weiteren Aspekt betrifft die vorliegende Erfindung ein Verfahren zur Herstellung eines erfindungsgemäßen elektrochemischen Sensors, umfassend die Schritte:
(a) Bereitstellen mindestens eines partikelförmigen Materials,
(b) Vermischen des partikelförmigen Materials mit mindestens einem Bindemittel und gegebenenfalls weiteren Substanzen,
(c) Verarbeiten des in Schritt (b) erhaltenen Gemisches zu einer Paste,
(d) Aufbringen der in Schritt (c) erhaltenen Paste auf einen Träger, umfassend eine Arbeitselektrode und eine Gegenelektrode, und Aushärten der auf den Träger aufgebrachten Paste,
(e) Aufbringen einer mindestens eine Ausnehmung umfassenden gaspermeablen Deckschicht auf den in Schritt (d) erhaltenen Träger, und
(f) gegebenenfalls Einbringen einer Schicht eines hydrophilen polymeren Füllmaterials in die Ausnehmung der in Schritt (e) auf den Träger aufgebrachten gaspermeablen Deckschicht.

Zum Zwecke der Herstellung der erfindungsgemäßen elektrochemischen Sensoren wird zumindest zur Herstellung der Paste gemäß Schritt (c) ein anorganisches oder organisches partikelförmiges Material mit mindestens einem anorganischen oder organischen Bindemittel, welche jeweils wie vorstehend definiert sind, und gegebenenfalls weiteren Substanzen vermischt und zu einer Paste verarbeitet. Nach Aufbringen der Paste auf einen Träger, umfassend eine Arbeitselektrode und eine Gegenelektrode, beispielsweise mittels Siebdruck oder mittels Rakeltechniken, und anschließendes Aushärten der Paste wird eine poröse, nicht-quellbare Gerüststruktur ausgebildet, welche als Elektrolytschicht in einem elektrochemischen Sensor gemäß der vorliegenden Erfindung verwendet werden kann.

Nach Aufbringen einer eine Ausnehmung umfassenden gaspermeablen Deckschicht auf den vorstehend beschriebenen Träger, welche beispielsweise mittels Siebdruck unter gleichzeitiger Abdeckung eines vorgegebenen Bereichs des Trägers erfolgen kann, und gegebenenfalls zusätzlichem Aufbringen einer Schicht eines hydrophilen polymeren Füllmaterials, welche vorzugsweise im Bereich der Ausnehmung der gaspermeablen Deckschicht auf den Träger bzw. die darauf befindliche Elektrolytschicht aufgebracht wird, kann ein elektrochemischer Sensor, wie er in vorliegender Anmeldung offenbart ist, erhalten werden. Das oben beschriebene Herstellungsverfahren erweist sich dabei insofern als vorteilhaft, da sowohl die Paste als auch die Elektroden mittels Siebdruck und damit in dünner und definierter Schichtstärke auf den Träger aufgebracht werden können.

Bevorzugt umfasst das erfindungsgemäße Verfahren ferner das Einbringen einer nicht-flüchtige Elektrolytbestandteile enthaltenden Flüssigkeit, z.B. eines flüssigen Elektrolyten, in die poröse, nicht-quellbare Gerüststruktur und anschließendes Abdampfen deren flüchtiger Bestandteile. Aus diesen nicht-flüchtigen Bestandteilen kann der flüssige Elektrolyt gebildet werden, welcher für die elektrisch leitende Verbindung zwischen der Arbeitselektrode und der Gegenelektrode in dem erfindungsgemäßen Sensor sorgt. Die Flüssigkeit kann hierbei unter Verwendung von Techniken, wie sie im Zusammmenhang mit der Erläuterung des elektrochemischen Sensors beschrieben sind, in die Poren der nicht-quellbaren Gerüststruktur eingebracht werden.

Alternativ können die nicht-flüchtigen Elektrolytbestandteile bereits vor Ausbildung der pörosen, nicht-quellbaren Gerüststruktur in ein Gemisch eingebracht werden, das mindestens ein partikelförmiges Material mit mindestens einem Bindemittel und gegebenenfalls weitere Substanzen enthält, wodurch auf einfache Weise eine gleichmäßige Verteilung der festen Bestandteile des Elektrolyten in der nach Aushärten des Gemisches erhaltenen porösen, nicht-quellbaren Gerüststruktur erzielt werden kann.

In einer bevorzugten Ausführungsform der Erfindung umfasst das erfindungsgemäße Verfahren das Einbringen der Flüssigkeit in eine Paste, wobei die Flüssigkeit der Paste insbesondere vor Aufbringen derselben auf einen geeigneten Träger zugesetzt werden kann. In dieser Ausführungsform wird eine Flüssigkeit zugegeben, welche die nicht-flüchtigen Elektrolytbestandteile vorzugsweise in einem mit dem Bindemittel mischbaren Lösungsmittel enthält. Dieses Lösungsmittel kann beispielsweise ein Glykol wie etwa Ethylenglykol, Propylenglykol oder ein Gemisch davon, das gegebenenfalls bis zu 20 % (v/v) Wasser enthalten kann, sein.

In noch einem weiteren Aspekt betrifft die vorliegende Erfindung ein Verfahren zur Bestimmung von in einem wässrigen Messmedium gelöstem Sauerstoff, umfassend die Schritte:
(a) Inkontaktbringen des wässrigen Messmediums mit einem erfindungsgemäßen elektrochemischen Sensor, und
(b) Bestimmen des in dem wässrigen Messmedium gelösten Sauerstoffs durch Messung eines von dem elektrochemischen Sensor erzeugten Signals.

Zur Bestimmung von Sauerstoff als gasförmigem Analyt kann der erfindungsgemäße elektrochemische Sensor in einer beliebigen Art und Weise ausgestaltet sein, welche einen Kontakt zwischen dem elektrochemischen Sensor und dem wässrigen Messmedium ermöglicht. So kann der Sensor beispielsweise als sensitiver Bestandteil einer Messzelle ausgebildet sein, in welche das den Sauerstoff enthaltende wässrige Messmedium eingebracht wird. Ferner kann der Sensor zusammen mit weiteren Sensoren, welche beispielsweise der Bestimmung unterschiedlicher "Point-of-Care"-Parameter dienen, in austauschbaren Messkammern (Sensor-Kassetten) ausgebildet sein.

Abhängig vom Vorhandensein oder/und der Menge des Analyten wird vom Sensor ein messbares Signal erzeugt. Der erfindungsgemäße elektrochemische Sensor ist vorzugsweise als amperometrischer Sensor ausgebildet, bei welchem als Messsignal der elektrische Strom unter Verwendung geeigneter Mittel ausgewertet bzw. ausgelesen wird.

Die Erfindung soll durch die nachfolgenden Figuren und Beispiele näher erläutert werden.

### Abbildungen

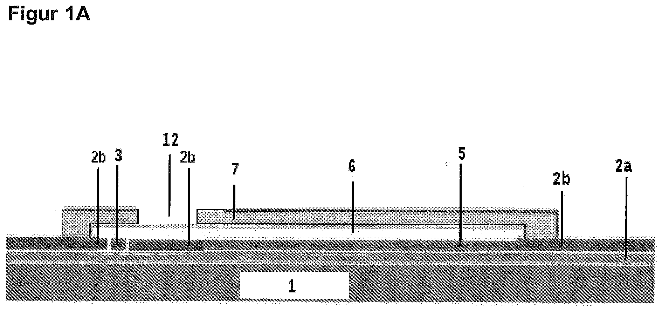

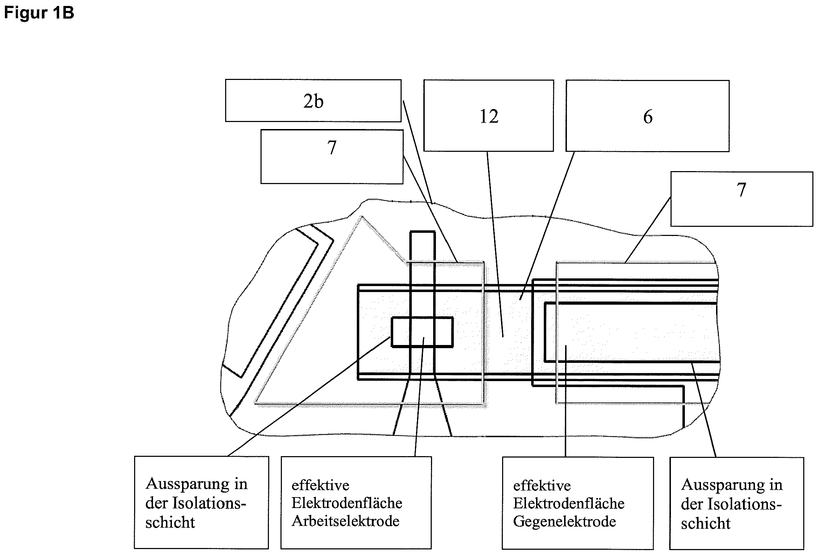

Fig. 1 a zeigt einen Schnitt durch den Kanalbereich in einem elektrochemischen pO₂-Sensor gemäß der vorliegenden Erfindung. Als Träger (1) dient ein mit einer Schicht eines Elektrosiolierlackes (2a) beschichtetes Metallplättchen, welche der Isolation der darauf angebrachten Elektroden gegenüber dem Metallplättchen dient. Auf dieser durchgehenden Isolierschicht (2a) ist in Teilbereichen (siehe Fig. 1b) eine weitere Isolierschicht (2b) aufgebracht, welche dazu vorgesehen ist, Störpotentiale/Ströme bei Flüssigkontakt zu vermeiden.

Im Messbereich ist ein Fenster ausgespart, in welchem die Arbeitselektrode (3), die Gegenelektrode (5), sowie die darüber befindliche poröse Elektrolytschicht (6) angeordnet sind. Die poröse Elektrolytschicht (6) verläuft längs des Messkanals und überdeckt die aktiven Flächen sowohl der Arbeitselektrode (3) als auch der Gegenelektrode (5) vollständig. Die gaspermeable Deckschicht (7) ist über der porösen Elektrolytschicht (6) angeordnet und weist zwischen der Arbeitselektrode (3) und der Gegenelektrode (5) eine als Spalt ausgebildete Ausnehmung (12) auf, welche die gaspermeable Deckschicht (7) in einen kathodenseitigen und einen anodenseitigen Teilbereich auftrennt.

In der in Fig. 1a dargestellten Ausführungsform überdeckt die gaspermeable Deckschicht (7) sowohl die Arbeitselektrode (3) als auch die Gegenelektrode (5) vollständig. Die Überdeckung der Arbeitselektrode (3) durch die gaspermeable Deckschicht (7) liegt hierbei in einem Bereich von etwa 200 µm bis etwa 600 µm, die Breite der Ausnehmung (12) in der gaspermeablen Deckschicht (7) üblicherweise im Bereich von etwa 100 µm bis etwa 400 µm.

Fig. 1b zeigt eine Aufsicht auf den Kanalbereich eines elektrochemischen pO₂-Sensors gemäß der vorliegenden Erfindung. In dieser Ausführungsform, in welcher ein Spalt (12) zwischen den beiden Teilbereichen der gaspermeablen Deckschicht (7) ausgebildet ist, ist die Oberfläche der Arbeitselektrode (3) vollständig, die Oberfläche der Gegenelektrode (5) nur teilweise von der gaspermeablen Deckschicht (7) überzogen. Die übrigen Bezugszeichen besitzen jeweils die in Fig. 1a angegebene Bedeutung.

Fig. 2a und Fig. 2b zeigen jeweils elektronenmikroskopische Aufnahmen der porösen Elektrolytschicht in einem elektrochemischen Sensor gemäß der vorliegenden Erfindung.

Fig. 3a zeigt den Gas-Nullpunkt [mmHg] eines Kollektivs elektrochemischer pO₂-Sensoren mit geschlossener Deckmembran gemäß Stand der Technik in Abhängigkeit von der Zeit nach dem erstmaligen Inkontaktbringen mit Kalibrationsflüssigkeit [h].

Fig. 3b zeigt den Gas-Nullpunkt [mmHg] eines Kollektivs erfindungsgemäßer elektrochemischer pO₂-Sensoren mit teiloffener Deckmembran in Abhängigkeit von der Zeit nach dem erstmaligen Inkontaktbringen mit Kalibrationsflüssigkeit [h].

Fig. 4a zeigt den Slope [nA] eines Kollektivs elektrochemischer pO₂-Sensoren mit geschlossener Deckmembran gemäß Stand der Technik in Abhängigkeit von der Zeit nach dem erstmaligen Inkontaktbringen mit Kalibrationsflüssigkeit [h].

Fig. 4b zeigt den Slope [nA] eines Kollektivs erfindungsgemäßer elektrochemischer pO₂-Sensoren mit teiloffener Deckmembran in Abhängigkeit von der Zeit nach dem erstmaligen Inkontaktbringen mit Kalibrationsflüssigkeit [h].

### Beispiele

### Beispiel 1: Herstellung einer siebdruckfähigen Paste

In ein verschließbares Glas wurden 12,0 g Ethylcellulose-Pulver N 50 und 192,0 g Terpineol eingewogen und das Gemisch bis zum vollständigen Lösen der Ethylcellulose 2 Wochen bei Raumtemperatur gerührt. Zur Herstellung einer Menge von 100 g an gebrauchsfertiger, siebdruckfähiger Paste wurden im Anschluss 25,6 g der erhaltenen Ethylcellulose-Lösung, 32,3 g Phenolharz PR 373, 1,0 g des Entschäumers Byk 051 (Fa. Byk Chemie), 5,0 g Benzylalkohol und 2,9 g Butyldiglykol in ein Becherglas eingewogen und dieses Gemisch intensiv gerührt. Zu diesem Gemisch wurden 43,2 g des Zeoliths LZ-Y 52 (Fa. Sigma-Aldrich, Produkt-Nr. 334448) hinzugefügt und der Ansatz bis zur homogenen Verteilung des Füllstoffes intensiv gerührt, wobei eine siebdruckfähige Paste erhalten wurde.

### Beispiel 2: Bestimmung der relativen Flüssigkeitsaufnahme einer nach Beispiel 1 hergestellten und ausgehärteten Paste

Zur Bestimmung der relativen Flüssigkeitsaufnahme einer nach Beispiel 1 hergestellten Paste wurden mehrere Objektträger (76 x 26 mm, ISO Norm 8038/I, Fa. Roth) auf einer Analysenwaage ausgewogen (m_{Träger}). Durch Aufbringen der nach Beispiel 1 hergestellten Paste auf die Objektträger unter Verwendung einer 80 µm Spiralabziehrakel wurden anschließend Prüfschichten auf die Objektträgern aufgetragen. Die aufgetragene Paste bedeckte eine rechteckige Fläche von ungefähr 60 x 18 mm. Da diese Fläche variabel ist, wurde sie erst nach dem Aushärten der Paste exakt bestimmt. 10-15 Minuten nach dem Auftragen der Paste wurden die Prüfkörper im Trockenschrank für 4 h bei 110 °C ausgehärtet. Anschließend wurden die Prüfkörper in einem Exsikkator (über CaO) bis zur Messung aufbewahrt. Die Objektträger mit der ausgehärteten Paste wurden erneut gewogen (m_{trocken}) und unmittelbar danach in jeweils etwa 40 ml Flüssigkeit eingetaucht, die sich in verschraubbaren Plastikdosen mit einem Volumen von 100 ml befand. Während des Messablaufs blieben die Dosen mit den eingetauchten Prüfkörpern verschlossen.

Zu definierten Zeitpunkten wurden die Prüfkörper aus den Dosen entnommen, abgetupft und gewogen, um die Menge an aufgenommener Flüssigkeit zu bestimmen. Die Messungen wurden abgebrochen, wenn die Menge an aufgenommener Flüssigkeit und damit die Masse (m_{feucht}) konstant blieb. Die Messungen wurden bei Raumtemperatur und Normaldruck durchgeführt. Aus den erhobenen Messwerten wurde für jeden Prüfkörper die relative Flüssigkeitsaufnahme als Mittelwert der entsprechenden Messwerte von mindestens 3 Prüfkörpern berechnet, mit einer Standardabweichung von maximal 1 %.

Die jeweilige relative Flüssigkeitsaufnahme wurde auf das jeweils zuvor bestimmte Trockengewicht der ausgehärteten Paste (jeweils abzüglich des Gewichts des Objektträgers) bezogen: (relative Flüssigkeitsaufnahme [%] = [(m_{feucht} - m_{Träger})/(m_{trocken} - m_{Träger}) - 1] * 100). Die Werte der relativen Flüssigkeitsaufnahme ändern sich entsprechend der Porosität der ausgehärteten Paste. Ausgehärtete Pasten mit einer unerwünscht hohen Porosität besitzen relative Flüssigkeitsaufnahmen zwischen 50 und 70 %, ausgehärtete Pasten mit einer unerwünscht niedrigen Porosität relative Flüssigkeitsaufnahmen von weniger als 20 %. Ausgehärtete Pasten, welche im Rahmen der vorliegenden Erfindung Anwendung finden, weisen eine Porosität auf, welche eine relative Flüssigkeitsaufnahme zwischen 20 Gew.-% und 50 Gew.-%, bevorzugt zwischen 26 Gew.-% und 44 Gew.-%, bewirkt.

### Beispiel 3: Herstellung eines Sensorhalbzeugs mit poröser Elektrolytschicht

Zur Herstellung eines Sensorhalbzeugs, welches als Bestandteil eines elektrochemischen Sensors gemäß der vorliegenden Erfindung verwendet werden kann, wurde die gemäß Beispiel 1 hergestellte Paste auf ein Sieb aufgetragen, mittels Siebdruck auf einen geeigneten Träger, umfassend eine Arbeitselektrode und eine Gegenelektrode, aufgebracht und bei erhöhter Temperatur zum Aushärten gebracht. Die Dicke der ausgehärteten Schicht betrug 10-20 µm.

### Beispiel 4: Herstellung eines für O₂-Sensoren geeigneten Sensorhalbzeugs mit poröser Elektrolytschicht

Zur Herstellung eines Sensorhalbzeugs, welches als Bestandteil eines elektrochemischen O₂-Sensors gemäß der vorliegenden Erfindung verwendet werden kann, wurden 0,310 g NaCl (p.a.) und 0,066 g Polyethylenglykol 6000 (zur Synthese) in 1,6 g entionisiertem Wasser gelöst. Die auf diese Weise erhaltene Lösung wurde anschließend mit einer Lösung von 0,900 g Glycerin (p.a.) in 23,15 g Ethylengykol versetzt und bis zum Erhalt einer klaren Lösung gerührt.

Zur Herstellung des Sensorhalbzeugs wurden 4,2 g der wässrigen Ethylenglykollösung in 100 g der nach Beispiel 1 hergestellten Paste eingerührt, woraufhin die den flüssigen Elektrolyten enthaltende Paste mittels Siebdruck auf einen geeigneten Träger aufgebracht und bei erhöhter Temperatur zum Aushärten gebracht wurde.

### Beispiel 5: Aufbringung einer teiloffenen, gaspermeablen Deckschicht auf das nach Beispiel 4 hergestellte Sensorhalbzeug

Die Aufbringung einer gaspermeablen Deckschicht, welche ihrerseits eine Ausnehmung aufweist, auf das gemäß Beispiele 4 hergestellte Sensorhalbzeug erfolgte mittels Siebdruck. Hierzu wurde eine 80%-ige Lösung von DELO-GUM® SI 480 (Fa. DELO) in n-Heptan mittels Siebdruck auf das Sensorhalbzeug aufgetragen und bei erhöhter Temperatur zum Aushärten gebracht.

Die Siebdruckstruktur wurde derart gewählt, dass eine spaltförmige Ausnehmung mit einer mittleren Breite von 300 ±100 µm erzeugt wurde, wobei der anodenseitige Kathodenrand 400 ± 100 µm vom kathodenseitigen Teilbereich der gaspermeablen Deckschicht überdeckt war und der kathodenseitige Anodenrand bündig mit dem anodenseitigen Teilbereich der gaspermeablen Deckschicht abschloss.

### Beispiel 6: Aufbringung einer Schicht eines hydrophilen polymeren Füllmaterials auf das nach Beispiel 5 hergestellte Sensorhalbzeug

Die Aufbringung einer optionalen Schicht eines hydrophilen polymeren Füllmaterials auf das gemäß Beispiel 5 hergestellte Sensorhalbzeug erfolgt beispielsweise durch Aufdispensieren. Hierzu wird beispielsweise eine 1%-ige Lösung eines hydrophilen Polyether-Polyurethan-Copolymers mit mindestens 50% Wasseraufnahme mittels einer Mikrodispensiervorrichtung in Form eines etwa 10 nl großen Tropfens in die Ausnehmung zwischen dem kathodenseitigen und dem anodenseitigen Teilbereich der gaspermeablen Deckschicht eingebracht und anschließend getrocknet.

## Patentansprüche

1. Elektrochemischer Sensor zur Bestimmung von in einem wässrigen Messmedium gelöstem Sauerstoff, wobei der Sensor als amperometrischer Sensor ausgebildet ist, umfassend:
(a) eine Arbeitselektrode (3) und eine Gegenelektrode (5), welche aus Silber oder einem Verbundmaterial auf Basis von Silber ausgebildet ist,
(b) eine zwischen der Arbeitselektrode (3) und der Gegenelektrode (5) angeordnete Elektrolytschicht (6), wobei die Elektrolytschicht (6) mindestens ein partikelförmiges Material und mindestens ein Bindemittel umfasst, welche zusammen eine poröse, nicht-quellbare Gerüststruktur ausbilden, und wobei die Poren der nicht-quellbaren Gerüststruktur zur Aufnahme eines flüssigen Elektrolyten vorgesehen sind oder diesen enthalten, und
(c) eine über der Elektrolytschicht angeordnete gaspermeable Deckschicht (7),
**dadurch gekennzeichnet,**
**dass** die gaspermeable Deckschicht (7) mindestens eine Ausnehmung (12) umfasst, welche einen Kontakt eines Teilbereichs der Elektrolytschicht mit dem wässrigen Messmedium ermöglicht.

2. Elektrochemischer Sensor nach Anspruch 1,
**dadurch gekennzeichnet,**
**dass** die Ausnehmung (12) eine Breite im Bereich von etwa 50 µm bis etwa 500 µm, bevorzugt im Bereich von etwa 100 µm bis etwa 300 µm, aufweist.

3. Elektrochemischer Sensor nach Anspruch 1 oder 2,
**dadurch gekennzeichnet,**
**dass** die Ausnehmung (12) spaltförmig oder fensterartig ausgebildet ist.

4. Elektrochemischer Sensor nach einem der Ansprüche 1 bis 3,
**dadurch gekennzeichnet,**
**dass** die Ausnehmung (12) zumindest teilweise über dem zwischen der Arbeitselektrode (3) und der Gegenelektrode (5) liegenden Bereich der Elektrolytschicht (6) angeordnet ist.

5. Elektrochemischer Sensor nach einem der Ansprüche 1 bis 4,
**dadurch gekennzeichnet,**
**dass** die Ausnehmung (12) mit einem hydrophilen polymeren Füllmaterial, insbesondere mit einem Polyether-Polyurethan-Copolymer oder mit Polyvinylpyrrolidon, verfüllt ist, wobei sich das hydrophile polymere Füllmaterial von dem Material unterscheidet, aus welchem die gaspermeable Deckschicht (7) gebildet ist.

6. Elektrochemischer Sensor nach einem der Ansprüche 1 bis 5,
**dadurch gekennzeichnet,**
**dass** die gaspermeable Deckschicht (7) die Arbeitselektrode (3) vollständig und die Gegenelektrode (5) zumindest teilweise überdeckt.

7. Elektrochemischer Sensor nach einem der Ansprüche 1 bis 6,
**dadurch gekennzeichnet,**
**dass** die gaspermeable Deckschicht (7) mindestens 90% der Gegenelektrode (5), bezogen auf deren Oberfläche, überdeckt.

8. Elektrochemischer Sensor nach einem der Ansprüche 1 bis 7,
**dadurch gekennzeichnet,**
**dass** die gaspermeable Deckschicht (7) ein polymeres Material, insbesondere ein Polysiloxan, ein Polysulfon oder/und Polytetrafluorethylen, umfasst.

9. Verfahren zur Herstellung eines elektrochemischen Sensors nach einem der Ansprüche 1 bis 8, umfassend die Schritte:
(a) Bereitstellen mindestens eines partikelförmigen Materials,
(b) Vermischen des partikelförmigen Materials mit mindestens einem Bindemittel und gegebenenfalls weiteren Substanzen,
(c) Verarbeiten des in Schritt (b) erhaltenen Gemisches zu einer Paste,
(d) Aufbringen der in Schritt (c) erhaltenen Paste auf einen Träger, umfassend eine Arbeitselektrode und eine Gegenelektrode, welche aus Silber oder einem Verbundmaterial auf Basis von Silber ausgebildet ist, und Aushärten der auf den Träger aufgebrachten Paste,
(e) Aufbringen einer mindestens eine Ausnehmung umfassenden gaspermeablen Deckschicht auf den in Schritt (d) erhaltenen Träger, und
(f) gegebenenfalls Einbringen einer Schicht eines hydrophilen polymeren Füllmaterials in die Ausnehmung der in Schritt (e) auf den Träger aufgebrachten gaspermeablen Deckschicht.

10. Verfahren zur Bestimmung von in einem wässrigen Messmedium gelöstem Sauerstoff, umfassend die Schritte:
(a) Inkontaktbringen des wässrigen Messmediums mit einem elektrochemischen Sensor nach einem der Ansprüche 1 bis 8, und
(b) Bestimmen des in dem wässrigen Messmedium gelösten Sauerstoffs durch Messung eines von dem elektrochemischen Sensor erzeugten Signals.

## Claims

1. Electrochemical sensor for determining the oxygen dissolved in an aqueous measuring medium, in which the sensor is designed as an amperometric sensor, comprising:
(a) a working electrode (3) and a counter electrode (5), which is formed of silver or a silver-based composite material,
(b) an electrolyte layer (6) arranged between the working electrode (3) and the counter electrode (5), in which the electrolyte layer (6) comprises at least one particulate material and at least one binding material, which together form a porous, non-swelling matrix structure, and in which the pores of the non-swelling matrix structure are provided to absorb a liquid electrolyte or contain this, and
(c) a gas-permeable cover membrane (7) arranged on top of the electrolyte layer, **characterised in that** the gas-permeable cover membrane (7) comprises at least one recess (12), which enables a contact of a portion of the electrolyte layer with the aqueous measuring medium.

2. Electrochemical sensor in accordance with claim 1, **characterised in that**
the recess (12) has a width in the range from some 50 µm to some 500 µm, preferably in the range from some 100 µm to some 300 µm.

3. Electrochemical sensor in accordance with claim 1 or 2, **characterised in that**
the recess (12) is designed to be shaped like a slit or a window.

4. Electrochemical sensor in accordance with one of claims 1 to 3, **characterised in that**
the recess (12) is arranged at least to some extent partially on top of the portion of the electrolyte layer (6) between the working electrode (3) and the counter electrode (5).

5. Electrochemical sensor in accordance with one of claims 1 to 4, **characterised in that**
the recess (12) is filled with a hydrophilic polymer filler material, in particular with a polyether-polyurethane copolymer or with polyvinylpyrrolidone, in which the hydrophilic polymer filler material differs from the material from which the gas-permeable cover membrane (7) is formed.

6. Electrochemical sensor in accordance with one of claims 1 to 9, **characterised in that**
the gas-permeable cover membrane (7) completely covers the working electrode (3) and at least partially covers the counter electrode (5).

7. Electrochemical sensor in accordance with one of claims 1 to 6, **characterised in that**
the gas-permeable cover membrane (7) covers at least 90% of the counter electrode (5), based on its surface area.

8. Electrochemical sensor in accordance with one of claims 1 to 7, **characterised in that**
the gas-permeable cover membrane (7) comprises a polymer material, in particular a polysiloxane, a polysulfone or/and polytetrafluorethylene.

9. Process for producing an electrochemical sensor in accordance with one of claims 1 to 8, comprising the following steps:
(a) Provision of at least one particulate material,
(b) Mixing of the particulate material with at least one binding material and with further substances if necessary,
(c) Processing the mixture obtained in step (b) into a paste,
(d) Application of the paste obtained in step (c) onto a support comprising a working electrode and a counter electrode, which is formed of silver or a silver-based composite material and curing of the paste applied to the support,
(e) Application of a gas-permeable cover membrane comprising at least one recess in the support obtained in step (d), and
(f) if necessary, insertion of a layer of a hydrophilic polymer filler material into the recess in the gas-permeable cover membrane applied to the support in step (e).

10. Process for determining the oxygen dissolved in an aqueous measuring medium, comprising the following steps:
(a) Bringing the aqueous measuring medium into contact with an electrochemical sensor in accordance with one of claims 1 to 8,
and
(b) Determining the oxygen dissolved in the aqueous measuring medium by measuring a signal produced by an electrochemical sensor.

## Revendications

1. Capteur électrochimique pour déterminer l'oxygène dissous dans un milieu de mesure aqueux, le capteur étant configuré comme un capteur ampérométrique, comprenant:
(a) une électrode de travail (3) et une contre-électrode (5), laquelle est constituée d'argent ou d'un matériau composite à base d'argent,
(b) une couche d'électrolyte (6) disposée entre l'électrode de travail (3) et la contre-électrode (5), la couche d'électrolyte (6) comprenant au moins une matière particulaire et au moins un liant, lesquels constituent ensemble une structure cadre poreuse non expansive, et les pores de la structure cadre non expansive étant prévus pour l'absorption d'un électrolyte aqueux ou contenant celui-ci, et
(c) une couche de couverture (7) perméable aux gaz agencée au-dessus de la couche d'électrolyte,
**caractérisé en ce que**
la couche de couverture (7) perméable aux gaz comprend au moins une cavité (12), laquelle permet le contact d'une partie de la couche d'électrolyte avec le milieu de mesure aqueux.

2. Capteur électrochimique selon la revendication 1, **caractérisé en ce que** la cavité (12) présente une largeur dans l'intervalle compris entre environ 50 µm et environ 500 µm, de préférence dans l'intervalle compris entre environ 100 µm et environ 300 µm.

3. Capteur électrochimique selon la revendication 1 ou 2, **caractérisé en ce que** la cavité (12) est en forme de fissure ou de fenêtre.

4. Capteur électrochimique selon l'une des revendications 1 à 3, **caractérisé en ce que**, la cavité (12) est agencée au moins partiellement au-dessus de la zone de la couche d'électrolyte (6) située entre l'électrode de travail (3) et la contre-électrode (5).

5. Capteur électrochimique selon l'une des revendications 1 à 4, **caractérisé en ce que** la cavité (12) est remplie d'une matière de charge polymère hydrophile, en particulier d'un copolymère polyéther-polyuréthane ou de polyvinylpyrrolidone, la matière de charge polymère hydrophile se distinguant de la matière constituant la couche de couverture (7) perméable aux gaz.

6. Capteur électrochimique selon l'une des revendications 1 à 5, **caractérisé en ce que** la couche de couverture (7) perméable aux gaz recouvre intégralement l'électrode de travail (3) et au moins partiellement la contre-électrode (5).

7. Capteur électrochimique selon l'une des revendications 1 à 6, **caractérisé en ce que** la couche de couverture (7) perméable aux gaz recouvre au moins 90% de la contre-électrode (5), sur base de la surface de celle-ci.

8. Capteur électrochimique selon l'une des revendications 1 à 7,
**caractérisé en ce que** la couche de couverture (7) perméable aux gaz comprend un matériau polymère, en particulier du polysiloxane, de la polysulfone et/ou du polytétrafluoroéthylène.

9. Procédé de fabrication d'un capteur électrochimique selon l'une des revendications 1 à 8, comprenant les étapes suivantes:
(a) procurer au moins une matière particulaire,
(b) mélanger la matière particulaire avec au moins un liant et éventuellement d'autres substances,
(c) traiter le mélange obtenu à l'étape (b) en une pâte,
(d) appliquer la pâte obtenue à l'étape (c) sur un support, comprenant une électrode de travail et une contre-électrode, laquelle est en argent ou en un matériau composite à base d'argent, et durcissement de la pâte appliquée au support,
(e) appliquer une couche de couverture perméable aux gaz comprenant au moins une cavité sur le support obtenu à l'étape (d), et
(f) éventuellement appliquer une couche d'une matière de charge polymère hydrophile dans la cavité de la couche de couverture perméable aux gaz appliquée à l'étape (e) sur le support.

10. Procédé de détermination de l'oxygène dissous en milieu de mesure aqueux, comprenant les étapes suivantes:
(a) mise en contact du milieu de mesure aqueux avec un capteur électrochimique selon l'une des revendications 1 à 8,
et
(b) détermination de l'oxygène dissous dans le milieu de mesure aqueux par la mesure d'un signal produit par le capteur électrochimique.
